(19) Europäisches Patentamt
European Patent Office
Office européen des brevets

(11) Veröffentlichungsnummer: **0 519 247 A2**

# (12) EUROPÄISCHE PATENTANMELDUNG

(21) Anmeldenummer: **92109045.2**

(51) Int. Cl.[5]: **C07C 209/36**

(22) Anmeldetag: **29.05.92**

(30) Priorität: **19.06.91 DE 4120192**

(43) Veröffentlichungstag der Anmeldung: **23.12.92 Patentblatt 92/52**

(84) Benannte Vertragsstaaten: **BE CH DE FR GB LI NL**

(71) Anmelder: **BASF Aktiengesellschaft**
**Carl-Bosch-Strasse 38**
**W-6700 Ludwigshafen(DE)**

(72) Erfinder: **Wolf, Hans-Josef, Dr.**
**Fichtestrasse 7**
**W-6701 Maxdorf(DE)**
Erfinder: **Goetz, Norbert, Dr.**
**Schoefferstrasse 25**
**W-6520 Worms 1(DE)**
Erfinder: **Kochanek, Wolfgang, Dr.**
**Freiheitsstrasse 57**
**W-6730 Neustadt(DE)**

## (54) Verfahren zur Herstellung von halogenierten Anilinen.

(57) Demgemäß wurde ein neues und verbessertes Verfahren zur Herstellung von halogenierten Anilinen der allgemeinen Formel I

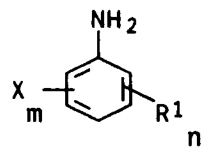

(I),

in der

- $R^1$ Wasserstoff, $C_1$- bis $C_8$-Alkyl, $C_3$- bis $C_8$-Cycloalkyl, $C_1$- bis $C_8$-Halogenalkyl, $C_1$- bis $C_8$-Hydroxyalkyl, $C_2$- bis $C_8$-Alkoxyalkyl, gegebenenfalls durch $C_1$- bis $C_4$-Alkyl, $C_1$- bis $C_4$-Alkoxy und/oder Halogen substituiertes Phenyl, $C(OR^2)_2$ und/oder $NR_2{}^2$
- $R^2$ $C_1$- bis $C_8$-Alkyl oder beide $R^2$ gemeinsam eine $C_2$- bis $C_5$-Alkylenkette,
- x Halogen,
- n 0 bis 4 und
- m 1 bis 5

bedeuten, mit der Maßgabe, daß n+m gleich 5 ist, durch Umsetzung von halogenierten Nitroaromaten der allgemeinen Formel II

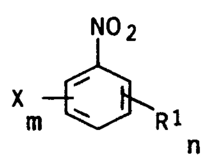

(II),

in der die Substituenten und die Indices die obengenannten Bedeutungen haben, bei Temperaturen von (-20) bis 100 °C, und Drücken von 0,1 bis 10 bar, indem man die Reaktion mit einer alkalischen Lösung von $Na_2Fe(CO)_4$ durchführt.

EP 0 519 247 A2

Die vorliegende Erfindung betrifft ein neues und verbessertes Verfahren zur Herstellung von halogenierten Anilinen durch Umsetzung von halogenierten Nitroaromaten bei Temperaturen von (-20) bis 100 °C und Drücken von 0, 1 bis 10 bar mit einer alkalischen $Na_2Fe(CO)_4$-Lösung.

Aus der J. Amer. Chem. Soc. 100, 3969 bis 3971 (1978) ist u.a. bekannt, Eisencarbonyl als Katalysator bei der Kohlenmonoxid-Reduktion von Nitroaromaten zu Anilinen zu verwenden. Neben langen Reaktionszeiten beobachtet man aber die allmähliche Zersetzung des Katalysators. Die Reaktion erfordert hohe Drucke und dementsprechend aufwendige Apparaturen. Zudem muß mit Kohlenmonoxidgas gearbeitet werden.

Die Reduktion aromatischer Nitroverbindungen mit Eisenpentacarbonyl und Alkalilauge ist aus der DE-PS 441 179 bekannt. Die Reaktion kann drucklos durchgeführt werden. Sie erfordert jedoch bei jeder Umsetzung die Verwendung des hochgiftigen $Fe(CO)_5$. Aus Bull. Chem. Soc. Jap. 48, 1478-1479 (1975) ist $KHFe(CO)_4$ in ethanolischer Lösung zur Reduktion einfacher substituierter Nitroaromaten bekannt. Es werden nur einfach chlorierte Nitroaromaten eingesetzt und der Reaktionsverlauf ist heftig. Die bei den Chloranilinen genannten Ausbeuten sind zudem größer als 100 %, weil offenbar die Reinigung der Produkte nicht gelingt. Weiterhin fällt bei der Darstellung des Reagenzes festes Kaliumcarbonat als Bodenkörper an und die Lösung des Reagenzes ist somit letztlich nicht homogen.

Der vorliegenden Erfindung lag daher die Aufgabe zugrunde, den zuvor genannten Nachteilen abzuhelfen.

Demgemäß wurde ein neues und verbessertes Verfahren zur Herstellung von halogenierten Anilinen der allgemeinen Formel I

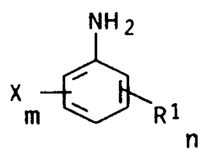

(I),

in der

- $R^1$ Wasserstoff, $C_1$- bis $C_8$-Alkyl, $C_3$- bis $C_8$-Cycloalkyl, $C_1$- bis $C_8$-Halogenalkyl, $C_1$- bis $C_8$-Hydroxyalkyl, $C_2$- bis $C_8$-Alkoxyalkyl, gegebenenfalls durch $C_1$- bis $C_4$-Alkyl, $C_1$- bis $C_4$-Alkoxy und/oder Halogen substituiertes Phenyl, $C(OR^2)_2$ und/oder $NR^2_2$
- $R^2$ $C_1$- bis $C_8$-Alkyl oder beide $R^2$ gemeinsam eine $C_2$- bis $C_5$-Alkylenkette,
- x Halogen,
- n 0 bis 4 und
- m 1 bis 5

bedeuten, mit der Maßgabe, daß n + m gleich 5 ist, durch Umsetzung von halogenierten Nitroaromaten der allgemeinen Formel II

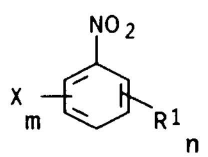

(II),

in der die Substituenten und die Indices die obengenannten Bedeutungen haben, bei Temperaturen von (-20) bis 100 °C, und Drücken von 0,1 bis 10 bar gefunden, welches dadurch gekennzeichnet ist, daß man die Reaktion mit einer alkalischen Lösung von $Na_2Fe(CO)_4$ durchführt.

Das erfindungsgemäße Verfahren läßt sich wie folgt durchführen:

Zu dem Nitroaromaten, vorzugsweise in einem inerten Lösungsmittel, wird eine alkalische Lösung von $Na_2Fe(CO)_4$ bei Temperaturen von (-20) bis +100 °C, bevorzugt 0 bis 80 °C, besonders bevorzugt 15 bis 45 °C und Drücken von 0,1 bis 10 bar, bevorzugt von 0,5 bis 2 bar, besonders bevorzugt unter Normaldruck (Atmosphärendruck) zugegeben, z.B. zugetropft, und die Reaktion durchgeführt.

Als alkalische Lösungen eignen sich wäßrige oder alkoholische Lösungen von Alkalimetallhydroxiden oder Erdalkalihydroxiden wie Lithiumhydroxid, Natriumhydroxid, Kaliumhydroxid, Rubidiumhydroxid, Cäsiumhydroxid, Calciumhydroxid und Magnesiumhydroxid, bevorzugt Kaliumhydroxid, Natriumhydroxid, Calciumhydroxid und Magnesiumhydroxid, besonders bevorzugt Natriumhydroxid, in Wasser oder einem $C_1$- bis $C_6$-Alkanol, wie Methanol, Ethanol, n-Propanol, iso-Propanol, n-Butanol, iso-Butanol, sec.-Butanol, tert.-

Butanol, n-Pentanol und Hexanol, bevorzugt Methanol, Ethanol, iso-Propanol und tert.-Butanol, besonders bevorzugt Methanol, Ethanol und iso-Propanol.

$Na_2Fe(CO)_4$ läßt sich analog DE-PS 441 179 herstellen bzw. z.B. durch Umsetzung von Eisenpentacarbonyl $Fe(CO)_5$ mit wäßrigem oder alkoholischem Natriumhydroxid bei Temperaturen von 0 bis 180 °C, bevorzugt von 15 bis 120 °C unter Atmosphärendruck (Normaldruck). Als Alkohole eignen sich die zuvorgenannten besonders.

Als inerte Lösungsmittel eignen sich z.B. polare aprotische oder protische organische Lösungsmittel wie Alkohole, z.B. $C_1$- bis $C_6$-Alkanol, wie Methanol, Ethanol, n-Propanol, iso-Propanol, n-Butanol, iso-Butanol, sec.-Butanol, tert.-Butanol, n-Pentenol und Hexanol, bevorzugt Methanol, Ethanol, iso-Propanol und tert.-Butanol, besonders bevorzugt Methanol, Ethanol und iso-Propanol.

Die Reaktion kann auch in einem Zweiphasensystem in Gegenwart eines Phasentransferkatalysatores durchgeführt werden. Als Lösungsmittel eignen sich Alkane, wie $C_5$- bis $C_{20}$-Alkane, z.B. Pentane, Hexane, Heptane, Octan oder aromatische Verbindungen wie Benzol, Toluol oder die Xylole.

Als Phasentransferkatalysatoren eignen sich Tetraalkylammoniumsalze oder Alkylarylammoniumsalze sowie Tetraalkylphosphoniumsalze oder Alkylarylphosphoniumsalze. Für die Umsetzung können übliche Glasapparaturen Verwendung finden. Eine besonders einfache Apparatur, bestehend aus Reaktionskolben, Rührvorrichtung sowie Flüssigkeitsdosierung für das Reagenz sind ausreichend. Diese geringen apparativen Anforderungen lassen den Einsatz der Methode auch im technischen Maßstab zu. Die Reaktion kann diskontinuierlich oder bevorzugt auch kontinuierlich durchgeführt werden.

Die Reinigung des Rohproduktes kann unmittelbar im Anschluß erfolgen. Die Filtration der Reaktionsmischung wird mit einer gängigen Filtrier- oder Absaugvorrichtung durchgeführt. Erleichtert wird der Prozeß, wenn man den Rohansatz zunächst mit einem inerten Feststoff wie z.B. Kieselgur oder Sand verrührt, bevor man die Filtration vornimmt. In das so erhaltene Filtrat leitet man zur Fällung des Ammoniumsalzes mit Vorteil gasförmigen Chlorwasserstoff ein, um das Hydrochlorid zu fällen. Auch andere Mineralsäuren in wäßriger Lösung oder reiner Form sind hierfür geeignet. Die Freisetzung des aromatischen Amins erfolgt mit basischen Anorganika, und zwar vorteilhaft mit wäßrigen Lösungen der Hydroxyde der Alkali- oder Erdalkalimetalle, wie LiOH, NaOH; KOH, RbOH, CsOH, $Mg(OH)_2$, $Ca(OH)_2$, bevorzugt Calciumhydroxid, Kaliumhydroxid oder Natriumhydroxid.

Die derart freigesetzten aromatischen Amine I können sich als eigene Phase abscheiden und lassen sich leicht abtrennen, oder man kann sie mit einem Lösungsmittel aus der wäßrigen Phase extrahieren. Als Lösungsmittel können u.a. Ether, Kohlenwasserstoffe oder halogenierte Kohlenwasserstoffe dienen. Die so gewonnene organische Phase kann mit Wasser alkalifrei gewaschen und über einem Trockenmittel von Wasser befreit werden.

Als Trockenmittel eignen sich Molekularsiebe oder anorganische Salze mit wasserbindenden Eigenschaften sein. Dabei sind bevorzugt Stoffe zu verwenden, die in Wasser eine neutrale oder alkalische Reaktion zeigen um Substanzverluste durch Oberflächenreaktion mit dem Amin zu vermeiden wie z.B. Calciumoxid, Magnesiumoxid und Calciumsulfat und Calciumchlorid.

Die beschriebene Reinigungsmethode liefert besonders reine Produkte, da die Produkte der unvollständigen Reduktion von Nitroaromaten unter den genannten Bedingungen keine Hydrohalogenide bilden.

Die Substituenten $R^1$, $R^2$, X und die Indices n und m in den Verbindungen I und II haben folgende Bedeutungen:

$R^1$ - unabhängig voneinander - Wasserstoff,

- $C_1$- bis $C_8$-Alkyl wie Methyl, Ethyl, n-Propyl, iso-Propyl, n-Butyl, iso-Butyl, sec.-Butyl, tert.-Butyl, n-Pentyl, iso-Pentyl, tert.-Amyl, n-Hexyl, iso-Hexyl, n-Heptyl, iso-Heptyl, n-Octyl und iso-Octyl, bevorzugt $C_1$- bis $C_4$-Alkyl wie Methyl, Ethyl, n-Propyl, iso-Propyl, n-Butyl, iso-Butyl, sec.-Butyl und tert.-Butyl, besonders bevorzugt Methyl und Ethyl,
- $C_3$- bis $C_8$-Cycloalkyl wie Cyclopropyl, Cyclobutyl, Cyclopentyl, Cyclohexyl, Cycloheptyl und Cyclooctyl, bevorzugt Cyclopropyl, Cyclopentyl, Cyclohexyl und Cyclooctyl,
- $C_1$- bis $C_8$-Halogenalkyl, bevorzugt $C_1$- bis $C_4$-Halogenalkyl, besonders bevorzugt $C_1$- bis $C_4$-Fluor, Chlor- und/oder Bromalkyl wie Fluormethyl, Difluormethyl, Trifluormethyl, Chlormethyl, Dichlormethyl, Trichlormethyl, Brommethyl, Dibrommethyl, Tribrommethyl,
- $C_1$- bis $C_8$-Hydroxyalkyl, bevorzugt $C_1$- bis $C_4$-Hydroxyalkyl wie Hydroxymethyl, 1-Hydroxyethyl, 2-Hydroxyethyl, 1-Hydroxy-n-propyl, 2-Hydroxy-n-propyl, 3-Hydroxy-n-propyl und 1-Hydroxymethyl-ethyl, besonders bevorzugt Hydroxymethyl, 1-Hydroxyethyl und 2-Hydroxyethyl,
- $C_2$- bis $C_8$-Alkoxyalkyl, bevorzugt $C_2$- bis $C_4$-Alkoxyalkyl wie Methoxymethyl, Ethoxymethyl, n-Propoxymethyl, iso-Propoxymethyl, n-Butoxymethyl, iso-Butoxymethyl, sec.-Butoxymethyl, tert.-Butoxymethyl, 1-Methoxy-ethyl und 2-Methoxy-ethyl, besonders bevorzugt Methoxymethyl und Ethoxymethyl,

- Phenyl,
- durch $C_1$- bis $C_4$-Alkyl und/oder $C_1$- bis $C_4$-Alkoxy und/oder Halogen substituiertes Phenyl, bevorzugt einfach bis dreifach substituiertes Methylphenyl, Ethylphenyl, n-Propylphenyl, einfach bis dreifach substituiertes Methoxyphenyl, Ethoxyphenyl, besonders bevorzugt einfach oder zweifach substituiertes Ethylphenyl, wie Methylphenyl, Ethoxyphenyl und Methoxyphenyl,

$R^2$ - $C_1$- bis $C_8$-Alkyl wie Methyl, Ethyl, n-Propyl, iso-Propyl, n-Butyl, iso-Butyl, sec.-Butyl, tert.-Butyl, n-Pentyl, iso-Pentyl, tert.-Amyl, n-Hexyl, iso-Hexyl, n-Heptyl, iso-Heptyl, n-Octyl und iso-Octyl, bevorzugt $C_1$- bis $C_4$-Alkyl wie Methyl, Ethyl, n-Propyl, iso-Propyl, n-Butyl, iso-Butyl, sec.-Butyl und tert.-Butyl, besonders bevorzugt Methyl und Ethyl,

x - Halogen, wie Fluor, Chlor, Brom und Jod, bevorzugt Fluor, Chlor und Brom,

n - die ganzen Zahlen von 0 bis 4, wie 0, 1, 2, 3 und 4, bevorzugt 0, 1, 2 und 3, besonders bevorzugt 1 und 2,

m - die ganzen Zahlen von 1 bis 5, wie 1, 2, 3, 4 und 5, bevorzugt 2, 3, 4 und 5,

mit der Maßgabe, daß die Summe aus n und m gleich 5 ist.

Geeignete halogenierte Aniline und ihre entsprechenden halogenierten Nitroaromaten der allgemeinen Formeln I und II sind z.B.

| halogenierte Aniline I | halogeniere Nitroaromaten II |
|---|---|
| 2-Chloranilin | 2-Chlor-nitrobenzol |
| 3-Chloranilin | 3-Chlor-nitrobenzol |
| 4-Chloranilin | 4-Chlor-nitrobenzol |
| 2-Bromanilin | 2-Brom-nitrobenzol |
| 3-Bromanilin | 3-Brom-nitrobenzol |
| 4-Bromanilin | 4-Brom-nitrobenzol |
| 2-Fluoranilin | 2-Fluor-nitrobenzol |
| 3-Fluoranilin | 3-Fluor-nitrobenzol |
| 4-Fluoranilin | 4-Fluor-nitrobenzol |
| 2,4-Difluoranilin | 2,4-Difluor-nitrobenzol |
| 2,4-Dichloranilin | 2,4-Dichlornitrobenzol |
| 2,4,5-Trifluoranilin | 2,4,5-Trifluornitrobenzol |
| 2,4,6-Trifluoranilin | 2,4,6-Trifluornitrobenzol |
| 2,3,4,5-Tetrachloranilin | 2,3,4,5-Tetrachlor-nitrobenzol |
| 2,3,4,5,-Pentachloranilin | 2,3,4,5,6-Pentachlornitrobenzol |

Die nach dem erfindungsgemäßen Verfahren herstellbaren halogenierten Aniline I sind wichtige Vorprodukte für Wirkstoffe im Pflanzenschutz und Pharmawirkstoffe (z.B. DE-OS 38 19 439 und EP-A-61 741).

Beispiele

Herstellung des Reagenzes

200 g $Fe(CO)_5$ werden in 800 g 25 %ige Natronlauge vorgelegt und unter kräftigem Rühren auf 65 °C erwärmt. Es setzt eine exotherme Reaktion ein und das Azeotrop Wasser/$Fe(CO)_5$ kocht bei 86 °C am Rückfluß. Wenn die exotherme Reaktion beendet ist, liegt eine homogene und klare gelbe Lösung vor, die 22 %ig an $Na_2Fe(CO)_4$ ist und unmittelbar für die Reduktion der Nitroaromaten eingesetzt werden kann.

Beispiel 1

Herstellung von 2-Bromanilin

In einem mit Stickstoff gespülten Kolben werden 15 g 2-Bromnitrobenzol (0,074 mol) in 100 ml Ethanol vorgelegt und dazu 65,7 g einer 22 %igen wäßrigen alkalischen Lösung von $Na_2Fe(CO)_4$ (0,067 mol) im Laufe einer Stunde zugetropft. Dabei erwärmt sich die Reaktionsmischung auf 30 °C. Nach beendeter Zugabe wird das Rohprodukt mit 1 g Kieselgur verrührt und über eine Nutsche abgesaugt. In das so erhaltene dunkle Filtrat wird bis zur Sättigung HCl-Gas eingeleitet. Die Lösung färbt sich gelb und 2-Bromaniliniumhydrochlorid fällt feinkristallin aus. Der Niederschlag wird abgesaugt und mit 15 ml n-Pentan nachgewaschen. Der Filterrückstand wird mit 25 ml 20 %ige Natronlauge versetzt und das sich ölig

abscheidende Produkt zweimal mit 30 ml n-Pentan extrahiert, die organische Phase mit 40 ml Wasser gewaschen und mit wasserfreiem Natriumsulfat getrocknet. Nach Entfernen des Lösungsmittels bleibt 11,9 g (93 %) 2-Bromanilin zurück; Reinheit (gaschromatographisch) 98 %.

Beispiel 2

Herstellung von 2,4-Difluoranilin

Analog Beispiel 1 wurden 15,9 g (0,1 mol) 2,4-Difluornitrobenzol mit 45 g einer 22 %eigen Lösung von $Na_2Fe(CO)_4$ (0,046 mol) umgesetzt. Man erhielt 10,85 g (84 %) 2,4-Difluoranilin.

Beispiel 3

Herstellung von 2,3,4,5-Tetrachloranilin

Analog Beispiel 1 wurden 5 g (19,16 mmol) 2,3,4,5-Tetrachlornitrobenzol mit 18,78 g einer 22 %igen Lösung von $Na_2Fe(CO)_4$ (19,16 mmol) umgesetzt. Man erhielt 4,34 g (99 %) 2,3,4,5-Tetrachloranilin.

**Patentansprüche**

1. Verfahren zur Herstellung von halogenierten Anilinen der allgemeinen Formel I

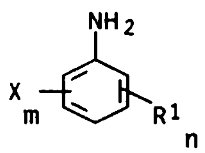

(I),

in der

$R^1$ Wasserstoff, $C_1$- bis $C_8$-Alkyl, $C_3$- bis $C_8$-Cycloalkyl, $C_1$- bis $C_8$-Halogenalkyl, $C_1$- bis $C_8$-Hydroxyalkyl, $C_2$- bis $C_8$-Alkoxyalkyl, gegebenenfalls durch $C_1$- bis $C_4$-Alkyl, $C_1$- bis $C_4$-Alkoxy und/oder Halogen substituiertes Phenyl, $C(OR^2)_2$ und/oder $NR^2_2$

$R^2$ $C_1$- bis $C_8$-Alkyl oder beide $R^2$ gemeinsam eine $C_2$- bis $C_5$-Alkylenkette,

x Halogen,

n 0 bis 4 und

m 1 bis 5

bedeuten, mit der Maßgabe, daß n + m gleich 5 ist, durch Umsetzung von halogenierten Nitroaromaten der allgemeinen Formel II

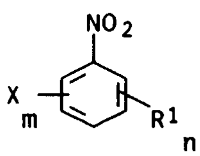

(II),

in der die Substituenten und die Indices die obengenannten Bedeutungen haben, bei Temperaturen von (-20) bis 100 °C, und Drücken von 0,1 bis 10 bar, dadurch gekennzeichnet, daß man die Reaktion in einer alkalischen Lösung von $Na_2Fe(CO)_4$ durchführt.

2. Verfahren zur Herstellung von halogenierten Anilinen nach Anspruch 1, dadurch gekennzeichnet, daß man als alkalische Lösung eine wäßrige Alkalihyroxidlösung verwendet.

3. Verfahren zur Herstellung von halogenierten Anilinen nach Anspruch 1, dadurch gekennzeichnet, daß man die halogenierten Nitroaromaten zum $Na_2Fe(CO)_4$ im Molverhältnis von 0,1:1 bis 2,17:1 einsetzt.